(19) Europäisches Patentamt
European Patent Office
Office européen des brevets

(11) Numéro de publication : **0 200 607**
**B1**

(12) **FASCICULE DE BREVET EUROPÉEN**

(45) Date de publication du fascicule du brevet : **05.04.89**

(51) Int. Cl.⁴ : **C 07 C129/16**

(21) Numéro de dépôt : **86400720.8**

(22) Date de dépôt : **03.04.86**

# (54) Nouveaux sels de chlorhexidine tensioactifs et compositions les contenant.

(30) Priorité : **05.04.85 FR 8505238**

(43) Date de publication de la demande : **05.11.86 Bulletin 86/45**

(45) Mention de la délivrance du brevet : **05.04.89 Bulletin 89/14**

(84) Etats contractants désignés : **AT BE CH DE FR GB IT LI LU NL SE**

(56) Documents cités :
**FR-A- 1 159 174**
**GB-A- 710 105**

(73) Titulaire : **PIERRE FABRE COSMETIQUE**
**125 rue de la Faisanderie**
**F-75116 Paris (FR)**

(72) Inventeur : **Navarro, Roger**
**Chemin des Farguettes Lambert**
**F-81100 Castres (FR)**

(74) Mandataire : **Ahner, Francis et al**
**CABINET REGIMBEAU 26, avenue Kléber**
**F-75116 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention, réalisée au Centre de Recherches Pierre Fabre Cosmétique, concerne de nouveaux sels de chlorhexidine tensioactifs présentant des propriétés antibactériennes et étant compatibles avec les dérivés de type anionique. Elle concerne également les compositions cosmétologiques, dermato-cosmétologiques et/ou médicamenteuses contenant lesdits sels de chlorhexidine.

Il est connu que le 1,6-di-(p-chlorophényl-biguanido)hexane (CHX), à l'état de base libre ou sous la forme de l'un de ses sels d'addition non toxiques, constitue un principe actif antibactérien doté d'une bonne efficacité pour éliminer la plaque dentaire et/ou pour en empêcher la formation. De la même façon cet antiseptique est de plus en plus utilisé pour ces mêmes propriétés, en dermatologie sous forme de solutions, de savons liquides ou sous forme de pains, de crèmes ou onguents, d'aérosols, etc., ou encore pour la stérilisation des lentilles de contact.

On a cependant constaté que ce principe actif utilisé dans certains excipients pouvait être fortement inhibé. Cet inconvénient a d'ailleurs amené de nombreux chercheurs à envisager diverses solutions permettant d'éviter ces problèmes qui résultent généralement de la présence de dérivés de type anionique, qui sont souvent associés à la chlorhexidine. C'est ainsi que FR-A-2 341 302 a préconisé un excipient bien particulier restant compatible avec ce principe actif antibactérien au sein d'une composition dentifrice.

On a tout d'abord constaté que le 1,6-di-(p-chlorophényl-biguanido)hexane qui se comportait comme un agent cationique, voyait son activité antibactérienne inhibée par tous les autres ingrédients anioniques largement utilisés dans le domaine de la cosmétologie, de la dermatologie, de l'odontostomatologie et de la désinfection cutanée. C'est ainsi que l'on a constaté que le digluconate de CHX était inhibé par les alkyls sulfate de sodium, les savons de sodium, etc.

Il s'est avéré de plus que parmi les ingrédients cationiques, non ioniques et amphotères il en existait un certain nombre qui inhibait l'activité antibactérienne de ce principe actif. Sans pour autant vouloir être lié par cette interprétation, la demanderesse estime que ces inhibitions, constatées au sein de ces trois dernières catégories d'ingrédients, sont très vraisemblablement dues à la formation de complexes ou de micelles pouvant modifier l'activité dudit principe actif.

Pour résoudre ces problèmes de formulation, liés aux incompatibilités avec les excipients, la présente invention a eu pour but de mettre au point des dérivés de la chlorhexidine compatibles avec les excipients anioniques.

Dans la technique antérieure on avait déjà proposé de rendre soluble la chlorhexidine base insoluble dans l'eau, en la combinant à l'acide gluconique pour former un digluconate de chlorhexidine. La partie de la molécule de ce sel portant la charge positive du cation est la plus grande et donne donc à cette molécule une tendance cationique. L'acide gluconique portant la charge négative est plus court et ne peut donc pas équilibrer la charge ionique de la molécule pour l'annuler.

Le but de cette invention est de trouver des acides pouvant être neutralisés par la chlorhexidine base et dont la longueur de chaîne puisse contrebalancer le « poids ionique » de cette base, et donner un sel pouvant affaiblir la tendance cationique de la chlorhexidine base.

Un autre but de cette invention est la mise au point de certains sels de chlorhexidine solubles dans l'eau pour pouvoir être utilisés, dans les applications classiques des sels de chlorhexidine déjà connus comme le digluconate, l'acétate, le chlorure, le saccharinate, etc.

Les sels conformes à la présente invention sont des sels de chlorhexidine résultant de la neutralisation de la chlorhexidine base par une fonction acide libre d'un composé tensioactif anionique ou amphotère choisi parmi le groupe comprenant :

a) les acides alkyl-éthoxycarboxyliques de formule :

$$R—O—CH_2—(CH_2—O—CH_2)_n—COOH$$

dans laquelle

R représente un groupe alkyle en $C_8$ à $C_{20}$, et

n = 0 à 20

b) les acides alkylaryl-éthoxycarboxyliques de formule :

$$R—Ar—O—CH_2—(CH_2—O—CH_2)_n—COOH$$

dans laquelle

R représente un groupe alkyle en $C_8$ à $C_{20}$,

Ar représente un radical p-phénylène, diphénylène ou naphtylène, et

n = 0 à 20

c) les acides N-alkyl-β-aminopropioniques de formule :

$$R—NH—CH_2—CH_2—COOH$$

dans laquelle R représente un radical alkyle en $C_8$ à $C_{20}$

d) les acides N-alkyl-β-iminodipropioniques de formule :

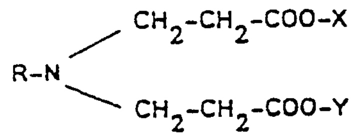

dans laquelle R représente un radical alkyle en $C_8$ à $C_{20}$, et l'un au moins des symboles X et Y désigne un atome d'hydrogène, l'autre pouvant être un métal alcalin ou un groupement ammonium

e) les acides alkyl-éthersulfoniques de formule

$$R—O—(CH_2—O—CH_2)_n—CH_2—CH_2—SO_3H$$

dans laquelle

R représente un radical alkyle en $C_8$ à $C_{20}$, et

n = 0 à 20

f) les acides alkylaryl-éthersulfonique de formule :

$$R—Ar\text{-}O—(CH_2—O—CH_2)_n—CH_2—CH_2—SO_3H$$

dans laquelle

R représente un radical alkyle en $C_8$ à $C_{20}$,

Ar représente un radical p-phénylène, diphénylène ou naphtylène, et

n = 0 à 20

g) l'acide lauroyl- ou palmitoyl-collagénique

h) l'acide caprylique hydroxyproline.

Ces nouveaux sels de chlorhexidine allient à la fois les propriétés antibactériennes de la chlorhexidine et les propriétés tensioactives de la chaîne acide de départ.

Ces nouveaux sels de chlorhexidine tensioactifs, peuvent être utilisés comme agents nettoyants, sous forme de dentifrice en remplacement des tensioactifs classiques du type alkyl sulfate de sodium, ou alkyl sarcosinate de sodium, etc., sous forme de savons liquides, de savons solides où ils remplaceront partiellement le tensioactif de base, ou sous forme d'émulsions où ce tensioactif pourra jouer le rôle d'émulgateur antiseptique.

Une autre caractéristique de cette invention est la mise au point de substances non tensioactives et non solubles dans l'eau qui auront pour avantage, si on les utilise avec un solvant approprié, tel que les alcools et les polyalcools, de se fixer, lors d'une application topique sur la peau et de pouvoir résister au lavage donc d'avoir une forte rémanence.

La présente invention s'étend également aux compositions contenant les sels précités à raison de 0,01 à 50 % en poids, de préférence de 0,01 à 10 %, par rapport au poids total de la composition qui peut en outre contenir d'autres solvants, en particulier les polyalcools, tels que les glycols par exemple le propylèneglycol.

C'est ainsi que l'invention concerne l'utilisation de nouveaux sels de CHX non solubles dans l'eau mais solubles dans la plupart des solvants utilisés en cosmétologie, en dermatologie et en pharmacie, et ayant une forte affinité pour les kératines, permettant une action de désinfection en surface des plaies, de la peau ou des muqueuses à traiter. Dans ce cadre on utilisera plus particulièrement :

— le lauroyl- ou palmitoyl-collagénate de CHX, et

— la capryl-hydroxyproline de chlorhexidine.

Ces sels sont obtenus à partir d'acide lauroyl- ou palmitoyl-collagénique, produit de condensation sur hydrolysat de collagène d'acide laurique ou palmitique, dont la fonction acide de l'hydrolysat de collagène reste libre.

Ces produits sont commercialisés par la Société RHONE-POULENC sous le nom de Lipacide LCO et PCO et par la Société MONTANOIR sous le nom de Montéine acide LCA.

L'invention concerne également des crèmes, émulsions fluides, solutions moussantes, lotions, savons, syndets, shampooings, dentifrices, aérosols, etc., ayant des propriétés antibactériennes et antifongiques, résultant de la présence des sels selon l'invention.

Il peut s'agir par exemple de compositions ayant des propriétés antiacnéiques, désinfectantes et/ou déodorantes. Il peut également s'agir de compositions de traitement du cheveu et du cuir chevelu, ayant des propriétés antipelliculaires et antiseptiques.

Les compositions de l'invention peuvent en outre être destinées au traitement de la cavité buccale. Elles peuvent aussi servir en tant qu'antiseptique de surface utilisable pour la désinfection de petites plaies et de brûlures. Il peut aussi s'agir de compositions à usage gynécologique dotées d'une action de surface plus ou moins profonde.

Lorsque les sels de CHX selon l'invention sont utilisés dans des compositions médicamenteuses du

type collyres, ils sont présents à raison d'une proportion plus faible de l'ordre de 0,01 à 0,5 % en poids du collyre. En revanche, dans des compositions de désinfection de matériel médico-chirurgical, les sels de CHX selon l'invention peuvent être présents à des teneurs beaucoup plus élevées pouvant atteindre jusqu'à environ 50 % en poids.

Les alkyl-éthoxycarboxylates de CHX peuvent être obtenus à partir des acides correspondants, par exemple ceux commercialisés sous la dénomination AKYPO® par la Société CHEMY.

Les N-alkyl-β-aminopropionate de CHX peuvent être obtenus à partir des acides correspondants de formule : $R—NH—CH_2CH_2COOH$ où R est une coupe acide gras de coprah, ou un mélange laurique-myristique, par exemple commercialisé sous le nom respectivement de Dériphat 151 C® et Dériphat 170 C® par la Société HENKEL.

Les N-alkyl-β-iminodipropionate de CHX sont par exemple obtenus à partir de l'acide correspondant de formule :

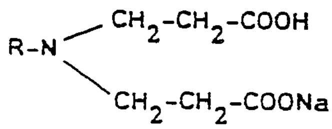

où R est le radical lauryl ; commercialisé sous le nom de Dériphat 160 C® par cette même Société HENKEL.

Enfin, la capryl-hydroxyproline de CHX peut être aisément obtenue à partir de l'acide caprylique hydroxyproline, produit de condensation sur hydroxyproline d'acide caprylique dont la fonction acide de l'hydroxyproline reste libre.

Ce produit est commercialisé par la Société RHONE-POULENC sous le nom de Lipacide $C_8CO$.

D'autres caractéristiques et avantages de la présente invention apparaîtront à la lecture de la description détaillée faite ci-après, notamment à l'aide des quelques exemples non limitatifs suivants.

## Exemple 1

Si on utilise l'alkyl éthoxycarboxylique acide dont le poids moléculaire est 624 (nom commercial Akypo RLM 100®), on constate après dosage qu'il faudra utiliser la formule suivante pour arriver à parfaite neutralisation

| | |
|---|---|
| — Akypo RLM 100® | 52,1 g |
| — CHX base | 18,2 g |
| — Propylène glycol | 29,7 g |
| | 100 g |

On pourra remarquer dans cette formule la présence de propylèneglycol, celui-ci jouant uniquement un rôle solvant pour la CHX base insoluble dans l'eau, ce solvant pouvant être remplacé par tout autre produit du type glycols, polyalcools ou choisi dans toutes autres familles (acétone, acétate d'éthyle, chlorure de méthylène, etc.). Il est bien évident que l'on choisira un solvant compatible avec les zones à traiter (peau-cheveu-muqueuses, etc.).

Dans cet exemple, le tensioactif de base, à savoir l'alkyl éthoxycarboxylique acide (AEC), est soluble dans l'eau ; il conduit à un sel de CHX soluble dans l'eau. Ce nouveau produit possède toutes les activités antibactériennes de la CHX base et toutes les propriétés tensioactives de l'alkyl éthoxycarboxylique acide neutralisé par une base quelconque, comme la soude.

Propriétés antibactériennes

Des expérimentations ont été conduites afin de vérifier si ce nouveau sel de CHX possédait une bonne activité antiseptique comparativement au digluconate de CHX, sel de CHX des plus connus et largement utilisé et ce comparativement à l'acide AEC, ce dernier pouvant avoir par lui-même des propriétés antiseptiques.

L'activité de ces trois produits a été testée sur quatre germes (gram⁻, gram— et levures) et il a été constaté que non seulement on obtenait une activité voisine de celle du digluconate de CHX, mais que de plus, cette activité était plus importante dans trois cas (voir tableau I).

(Voir Tableau I page 5)

Tableau I
Propriétés antibactériennes

| | Staphylocoque aureus | Escherichia coli ATCC 10536 | Pseudomonas aeruginosa A 22 | Candida albicans |
|---|---|---|---|---|
| Digluconate de CHX | 0,12 g/ml | 0,06 g/ml | 16 g/ml | 31,2 g/ml |
| AEC acide | 1,56 mg/ml | 12,5 mg/ml | 6,2 mg/ml | 50 mg/ml |
| AEC de CHX | 0,56 g/ml | 0,035 g/ml | 2,8 g/ml | 5,6 g/ml |

Pouvoir moussant

Le pouvoir moussant de l'acide éthoxycarboxylique et de l'éthoxycarboxylate de CHX a été vérifié, et ce comparativement.

A cet effet, on prépare une solution de tensioactif à 10 % de matière active. On prélève 50 ml de cette solution que l'on introduit dans une éprouvette de 100 ml (diamètre intérieur 26 mm et hauteur 235 mm). On bouche hermétiquement cette éprouvette et on la soumet à 10 retournements successifs pour provoquer la formation de mousse. Cette manipulation se fait à 23 °C (température de la solution).

Pour l'acide éthoxycarboxylique on obtient 26 ml de mousse.

Pour l'éthoxycarboxylate de CHX on obtient 16 ml de mousse.

Conclusion : L'éthoxycarboxylate de CHX a un pouvoir moussant inférieur à celui de la forme non neutralisée, mais il reste cependant assez important pour pouvoir être considéré comme un tensioactif avec toutes ses propriétés.

On peut penser que cette différence de pouvoir moussant est apportée par le solvant propylèneglycol.

Tolérance : On s'est contenté de vérifier la bonne tolérance cutanée et oculaire du tensioactif acide éthoxycarboxylique et du nouveau sel de CHX de cet exemple, pour bien démontrer la parfaite inocuité de ce nouveau produit.

— tolérance cutanée cobaye

On voit d'après ces résultats que cette tolérance est meilleure sur ce test pour le nouveau sel de CHX que pour l'acide éthoxycarboxylique. La différence d'épaisseur de pli de peau mesuré le 4$^{e}$ jour d'application quotidienne est de 0,23 contre 1,2 mm, l'érythème de 1,4 contre 4.

— tolérance cutanée lapin

(Technique J.O. du 21/04/71 et 20/10/73)

Même performance pour le sel de CHX, l'indice d'irritation primaire est de 0,25 contre 0,75 pour la forme acide commercialisée.

— tolérance oculaire lapin

(Technique J.O. du 21/04/71 et 20/10/73)

Sur ce test nous trouvons un résultat inversé, la forme acide se trouve être la mieux tolérée.

En conclusion de ces tests de tolérance, on peut dire que l'AEC de CHX et l'acide EC sont des produits qui peuvent se comporter de façon différente en fonction des tests utilisés, mais ils restent cependant tous deux dans les normes de tolérance pour des matières premières testées à 10 % en solution dans l'eau.

Toxicité des nouveaux sels de CHX

La toxicité de ce nouveau sel de CHX a été vérifiée comparativement au tensioactif sous forme acide de départ, au tensioactif neutralisé par l'ion sodium, à la CHX base et à l'association tensioactif neutralisé par l'ion sodium + CHX base, car il est bien connu que la toxicité d'une molécule peut être potentialisée par la présence d'un tensioactif.

Deux tests ont été utilisés :

1. l'évaluation de la DL 50 par voie orale,
2. l'évaluation de la DL 50 par voie intra-péritonéale.

(résultats : voir tableau II)

Comme on peut le voir sur ce tableau, les DL 50 sont peu ou pas modifiées, si on compare la forme acide et les formes salifiées par CHX et Na. La toxicité de l'alkyl éthoxycarboxylate de CHX est celle du tensioactif de départ.

La CHX base elle-même n'est pratiquement pas toxique.

Tableau II

Etude de toxicité

| PRODUIT TESTE | DL 50 voie orale en g/kg | DL 50 voie intrapéritonéale en g/kg |
|---|---|---|
| Acide alkyléthoxycarboxylique | 2,5 < DL 50 < 5 | 0,5 |
| Alkyl éthoxycarboxylate de CHX | 2,5 < DL 50 < 5 | 0,125 < DL 50 < 0,25 |
| Alkyl éthoxycarboxylate de sodium | 7,5 | 0,75 < DL 50 < 1 |
| chlorhexidine base | pas de mortalité | 0,047 < DL 50 < 0,1 |
| Association : Alkyl éthoxycarboxylate de sodium + CHX base | 2,5 < DL 50 < 5 | 0,125 < DL 50 < 0,25 |

Activité antimicrobienne de l'alkyl éthoxycarboxylate de CHX en présence de tensioactifs anioniques

La mise au point de ce nouveau type de sel de chlorhexidine ayant pour but de conduire à des produits chimiques compatibles avec les tensioactifs, principalement anioniques, nous avons dans ce test vérifié cette activité :

Détermination de l'activité antiseptique par une méthode de dilutions en milieu liquide, selon Peterson, Lance R., Denny, Aiken E., Gerding, Dale N. and Hall, Wendell H. : « determination of tolerance to antibiotic bactericidal on activity on Kivby-Baver susceptibility plates Am. J. Clin. Pathol. 74 : 645-650, 1980.

Le but de la méthode est de déterminer la plus petite quantité d'un produit X qui exerce sur $10^6$ cellules d'une flore bactérienne donnée une activité antiseptique de type bactériostatique ou bactéricide.

La méthode de dilution en milieu liquide permet de déterminer la CMI (concentration minimale inhibitrice-bactériostase) et la CMB (concentration minimale léthale-bactéricidie).

1. Matériel — milieu de culture :

— pipettes de 1 ml graduées au 1/100 en polystyrène cristal stériles,
— pipettes Pasteur stériles,
— flacons en verre stériles de 30 ml, 100 ml,
— tubes à essai en verre PYREX stériles 65 × 13 mm, 160 × 16 mm, 25 × 200 mm et 18 × 180 mm,

— boîtes de Pétri, polystyrène cristal stériles de 90 mm de diamètre,

— lumétron, colorimètre réf. 401,

— les milieux de culture liquides et gélosés sont adaptés à la croissance de l'espèce de microorganisme en test Pseudomonas aeruginosa. Un témoin de fertilité des milieux liquides et gélosés est réalisé à chaque expérimentation.

2. Préparation de la solution mère d'un produit X à analyser

Les différents produits testés sont des solutions aqueuses d'alkyl éthoxycarboxylate de chlorhexidine (3 %) et contenant un pourcentage variable de tensioactifs anioniques. Les solutions mères sont des dilutions au 1/10$^{e}$ des solutions précédentes.

3. Préparation de l'inoculum

— repiquage des microorganismes

Pseudomonas aeruginosa est ensemencé en stries sur une pente de gélose Tryptone Soya (CM 131 OXOID). On incube de 18 à 24 heures à 32-35 °C.

— préparation de l'inoculum

Pour le Pseudomonas aeruginosa, une transmission de 90 % représente une valeur de $2 \cdot 10^{8}$ germes/ml donc une dilution au 1/100$^{e}$ est nécessaire.

4. Détermination de la CMI et de la CMB

On prépare sur un portoir une série de tubes à hémolyse 65 × 13 mm stériles.

Chaque tube reçoit 1 ml de milieu de culture liquide dont la composition est adaptée à la croissance du germe (bouillon BN pour Pseudomonas aeruginosa).

Le tube reçoit 1 ml de la solution mère du produit à analyser. On homogénéise puis effectue des passages successifs au 1/2 jusqu'au dernier tube. Le volume dans tous les tubes est alors de 1 ml.

Toutes les dilutions sont ensemencées avec 1 ml de milieu de culture titrant $2 \cdot 10^{6}$ germes par ml. Dans chaque tube on a alors $1 \cdot 10^{6}$ germes par ml.

Un tube sans antiseptique permet d'effectuer un dénombrement des microorganismes effectivement introduits dans le test et permet de s'assurer de la vitalité de la souche.

Dénombrement par la méthode de dilution-incorporation et incubation à l'étuve pendant 48 heures à 37 °C.

Lecture

La concentration minimale inhibitrice est la plus petite quantité de produit qui empêche le développement microbien (jugé à l'œil) ; elle est exprimée en microgramme de chlorhexidine base par millilitre.

La concentration minimale bactéricide CMB est déterminée par sub-culture sur milieu neutralisant de tous les tubes qui ne montrent pas de développement microbien à l'œil ; elle est exprimée comme la CMI.

5. Résultats expérimentaux

— tests effectués sur Pseudomonas aeruginosa A 22 à une concentration de $10^{6}$ germes/ml ;

— résultats exprimés en microgramme de chlorhexidine base par millilitre.

(Voir tableau III page 8)

Tableau III
Activité antimicrobienne

| PRODUITS TESTES | CMI | CMB |
|---|---|---|
| Digluconate de chlorhexidine | 8,75 | 8,75 |
| Alkyl éthoxycarboxylate de chlorhexidine | 8 | 8 |
| Alkyl éthoxycarboxylate de chlorhexidine + 4 % de Alkyl éthersulfate de sodium et magnésium (TEXAPON ASV)® | 8 | > 510 |
| Alkyl éthoxycarboxylate de chlorhexidine + 5 % de Lauryl sulfate de triéthanolamine hydrolysat de protéines animales (LIPOPROTEOL LK et LCO)® | 8 | 8 |
| Alkyl éthoxycarboxylate de chlorhexidine + 5 % de Undécylénate de potassium hydrolysat de protéines animales (MAYPON UD)® | 8 | 8 |

Exemple 2

Si on utilise l'acide N-alkyl-β-aminopropionique dont le poids moléculaire st voisin de 272,2 (nom commercial Dériphat® 151 C), on constate après dosage qu'il faudra utiliser la formule suivante pour arriver à parfaite neutralisation :

| | |
|---|---|
| — Dériphat 151 C® | 50 g |
| — CHX base | 17 g |
| — Propylèneglycol | 43 g |

Toutes les remarques faites à propos de l'exemple 1 sont valables ici.

Exemple 3

Cet exemple a permis de découvrir un sel de CHX insoluble dans l'eau qui, comme on le verra plus loin, présente un avantage très particulier.

A partir de l'acide cocoyl collagénique (Montéine acide LCA®), on utilisera la formule suivante :

| | |
|---|---|
| — Montéine acide LCA | 50 g |
| — CHX base | 25,25 g |
| — Propylèneglycol | 24,75 g |

Dans ce cas précis, l'acide cocoyl collagénique est insoluble dans l'eau, sa neutralisation par la CHX base, elle-même insoluble dans l'eau, doit donc se faire en milieu propylèneglycol. Le sel obtenu est

insoluble dans l'eau donc le produit obtenu ne peut jouer un rôle de tensioactif. Cependant, l'insolubilité dans l'eau de ce sel de CHX présente un autre avantage. Il est soluble dans l'alcool éthylique et dans les hydrocarbures chloro-fluorés, d'où la possibilité de fabriquer des solutions ou des aérosols vecteurs de CHX fixée sur acide cocoyl collagénique dont les propriétés principales sont :

— fixation sur la peau comme tous les dérivés protéiques,
— produit non lavable à l'eau, donc forte substantivité,
— propriétés antiseptiques de la CHX.
On obtiendra les mêmes résultats en utilisant

Exemple 4

Les lipacides LCO, PCO et $C_8CO$

| | |
|---|---|
| — Lipacide LCO | 25 g |
| — CHX | 22 g |
| — Propylèneglycol | 53 g |
| — Lipacide PCO | 25 g |
| — CHX | 13,25 g |
| — Propylèneglycol | 61,75 g |
| — Lipacide $C_8CO$ | 25 g |
| — CHX | 25,25 g |
| — Propylèneglycol | 49,75 g |

Tous ces dérivés de type alkyl polypeptide ont des propriétés tensioactives s'ils sont neutralisés par des alcalis (sodium, triéthanolamine) et peuvent donc être utilisés comme nettoyant dans les shampooings.

Neutralisés par la CHX, on obtient des sels non solubles dans l'eau aux propriétés déjà décrites dans l'exemple 3.

**Revendications**

1. Sels de chlorhexidine dotés de propriétés antibactériennes et tensioactives, caractérisés en ce qu'ils résultent de la neutralisation de la chlorhexidine base par une fonction acide libre d'un composé tensioactif anionique ou amphotère choisi parmi le groupe comprenant :

a) les acides alkyl-éthoxycarboxyliques de formule :

$$R—O—CH_2—(CH_2—O—CH_2)_n—COOH$$

dans laquelle :
R représente un groupe alkyle en $C_8$ à $C_{20}$, et
n = 0 à 20

b) les acides alkylaryl-éthoxycarboxyliques de formule :

$$R—Ar—O—CH_2—(CH_2—O—CH_2)_n—COOH$$

dans laquelle
R représente un groupe alkyle en $C_8$ à $C_{20}$,
Ar représente un radical p-phénylène, diphénylène ou naphtylène, et
n = 0 à 20

c) les acides N-alkyl-β-aminopropioniques de formule :

$$R—NH—CH_2—CH_2—COOH$$

dans laquelle R représente un radical alkyle en $C_8$ à $C_{20}$

d) les acides N-alkyl-β-iminodipropioniques de formule :

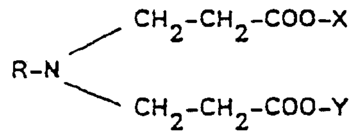

dans laquelle R représente un radical alkyle en $C_8$ à $C_{20}$, et l'un au moins des symboles X et Y désigne un atome d'hydrogène, l'autre pouvant être un métal alcalin ou un groupement ammonium

e) les acides alkyl-éthersulfoniques de formule

$$R—O—(CH_2—O—CH_2)_n—CH_2—CH_2—SO_3H$$

dans laquelle

R représente un radical alkyle en $C_8$ à $C_{20}$, et

n = 0 à 20

f) les acides alkylaryl-éthersulfonique de formule :

$$R—Ar—O—(CH_2—O—CH_2)_n—CH_2—CH_2—SO_3H$$

dans laquelle

R représente un radical alkyle en $C_8$ à $C_{20}$,

Ar représente un radical p-phénylène, diphénylène ou naphtylène, et

n = 0 à 20

g) l'acide lauroyl- ou palmitoyl-collagénique

h) l'acide caprylique hydroxyproline.

2. Les alkyl-éthoxycarboxylates de chlorhexidine selon la revendication 1.

3. Les alkylaryl-éthoxycarboxylates de chlorhexidine selon la revendication 1.

4. Les N-alkyl-β-aminopropionates de chlorhexidine selon la revendication 1.

5. Les N-alkyl-β-imino-dipropionates de chlorhexidine selon la revendication 1.

6. Le lauroyl-collagénate de chlorhexidine.

7. Le palmitoyl-collagénate de chlorhexidine.

8. La capryl-hydroxyproline de chlorhexidine.

9. Les alkyl-éthersulfonates de chlorhexidine.

10. Les alkylaryl-éthersulfonates de chlorhexidine.

11. Compositions cosmétologiques, dermato-cosmétiques et/ou médicamenteuses contenant au moins un sel de chlorhexidine selon l'une des revendications 1 à 5, associé à un solvant compatible.

12. Compositions selon la revendication 11, caractérisées en ce que le ou les sels de chlorhexidine sont présents à raison de 0,01 à 50 % en poids, de préférence de 0,01 à 10 %, par rapport au poids de la composition totale.

13. Compositions selon l'une des revendications 11 et 12, caractérisées en ce que ledit solvant est un polyalcool, en particulier un glycol tel que le propylèneglycol.

**Claims**

1. Chlorhexidine salts endowed with antibacterial and surfactant properties, characterized in that they result from the neutralization of chlorhexidine base by a free acid group of an anionic or amphoteric surfactant compound chosen from the group comprising :

a) alkylethoxycarboxylic acids of formula :

$$R—O—CH_2—(CH_2—O—CH_2)_n—COOH$$

in which

R denotes a $C_8$ to $C_{20}$ alkyl group, and

n = 0 to 20

b) alkylarylethoxycarboxylic acids of formula :

$$R—Ar—O—CH_2—(CH_2—O—CH_2)_n—COOH$$

in which

R denotes a $C_8$ to $C_{20}$ alkyl group,

Ar denotes a p-phenylene, diphenylene or naphthylene radical, and

n = 0 to 20

c) N-alkyl-β-aminopropionic acids of formula :

$$R—NH—CH_2—CH_2—COOH$$

in which R denotes a $C_8$ to $C_{20}$ alkyl radical

d) N-alkyl-β-iminodipropionic acids of formula :

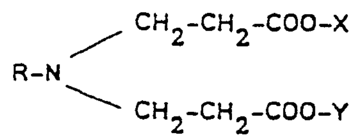

in which R denotes a $C_8$ to $C_{20}$ alkyl radical, and at least one of the symbols X and Y denotes a hydrogen atom, it being possible for the other to be an alkali metal or an ammonium group

e) alkyl ether sulphonic acids of formula :

$$R—O—(CH_2—O—CH_2)_n—CH_2—CH_2—SO_3H$$

in which

R denotes a $C_8$ to $C_{20}$ alkyl radical, and

n = 0 to 20

f) alkylaryl ether sulphonic acids of the formula :

$$R—Ar—O—(CH_2—O—CH_2)_n—CH_2—CH_2—SO_3H$$

in which

R denotes a $C_8$ to $C_{20}$ alkyl radical,

Ar denotes a p-phenylene, diphenylene or naphthylene radical, and

n = 0 to 20

g) lauroyl- or palmitoylcollagenic acid

h) hydroxyproline-caprylic acid.

2. The chlorhexidine alkylethoxycarboxylates according to Claim 1.
3. The chlorhexidine alkylarylethoxycarboxylates according to Claim 1.
4. The chlorhexidine N-alkyl-β-aminopropionates according to Claim 1.
5. The chlorhexidine N-alkyl-β-iminodipropionates according to Claim 1.
6. Chlorhexidine lauroylcollagenate.
7. Chlorhexidine palmitoylcollagenate.
8. Chlorhexidine caprylhydroxyproline.
9. Chlorhexidine alkyl ether sulphonates.
10. Chlorhexidine alkylaryl ether sulphonates.
11. Cosmetological, dermato-cosmetic and/or medicinal compositions containing at least one chlorhexidine salt according to one of Claims 1 to 5, in combination with a compatible solvent.
12. Compositions according to Claim 11, characterized in that the chlorhexidine salt or salts is/are present in the proportion of 0.01 to 50 % by weight, and preferably 0.01 to 10 %, relative to the weight of the whole composition.
13. Compositions according to one of Claims 11 and 12, characterized in that the said solvent is a polyhydric alcohol, especially a glycol such as propylene glycol.

**Patentansprüche**

1. Chlorhexidinsalze mit antibakteriellen- und oberflächenaktiven Eigenschaften, dadurch gekennzeichnet, daß sie resultieren aus der Neutralisation der Chlorhexidin-Base mit einer freien Säurefunktion einer anionischen oder amphoteren oberflächenaktiven Verbindung, ausgewählt aus der Gruppe, die umfaßt :

a) die Alkyl-ethoxycarbonsäuren der Formel :

$$R—O—CH_2—(CH_2—O—CH_2)_n—COOH$$

worin

R eine Alkylgruppe mit $C_8$ bis $C_{20}$ darstellt und

n = 0 bis 20

b) die Alkylaryl-ethoxycarbonsäuren der Formel :

$$R—Ar—O—CH_2—(CH_2—O—CH_2)_n—COOH$$

worin

R eine Alkylgruppe mit $C_8$ bis $C_{20}$ darstellt,

Ar einen p-Phenylen-, Diphenylen- oder Naphthylen-Rest darstellt und

n = 0 bis 20

c) die N-Alkyl-β-aminopropionsäuren der Formel :

$$R—NH—CH_2—CH_2—COOH$$

worin R einen Alkylrest mit $C_8$ bis $C_{20}$ darstellt,

d) die N-Alkyl-β-iminodipropionsäuren der Formel :

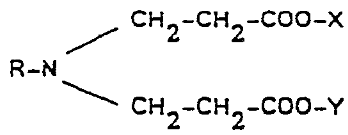

worin R einen Alkylrest mit $C_8$ bis $C_{20}$ darstellt und mindestens eines der Symbole X und Y ein Wasserstoffatom bedeutet, wobei das andere ein Alkalimetall oder eine Ammoniumgruppe sein kann

e) die Alkyl-ethersulfonsäuren der Formel :

$$R—O—(CH_2—O—CH_2)_n—CH_2—CH_2—SO_3H$$

worin

R einen Alkylrest mit $C_8$ bis $C_{20}$ bedeutet, und

n = 0 bis 20

f) die Alkylaryl-ethersulfonsäuren der Formel :

$$R—Ar—O—(CH_2—O—CH_2)_n—CH_2—CH_2—SO_3H$$

worin :

R einen Alkylrest mit $C_8$ bis $C_{20}$ darstellt,

Ar einen p-Phenylen-, Diphenylen- oder Naphthylen-Rest bedeutet, und

n = 0 bis 20

g) Lauroyl- oder Palmytoyl-collagensäure

h) Caprylsäure-Hydroxyprolin

2. Die Alkyl-ethoxycarboxylate von Chlorhexidin nach Anspruch 1.

3. Die Alkylaryl-ethoxycarboxylate von Chlorhexidin nach Anspruch 1.

4. Die N-Alkyl-β-aminopropionate von Chlorhexidin nach Anspruch 1.

5. Die N-Alkyl-β-imino-dipropionate von Chlorhexidin nach Anspruch 1.

6. Das Lauroyl-collagenat von Chlorhexidin.

7. Das Palmitoyl-collagenat von Chlorhexidin.

8. Das Capryl-hydroxyprolin von Chlorhexidin.

9. Die Alkyl-ethersulfonate von Chlorhexidin.

10. Die Alkylaryl-ethersulfonate von Chlorhexidin.

11. Kosmetologische, haut-kosmetische Zusammensetzungen und/oder Arzneimittel-Zusammensetzungen, enthaltend mindestens ein Chlorhexidinsalz nach einem der Ansprüche 1 bis 5, zusammen mit einem verträglichen Lösungsmittel.

12. Zusammensetzungen nach Anspruch 11, dadurch gekennzeichnet, daß das oder die Chlorhexidinsalze in einer Menge von 0,01 bis 50 Gew.-% vorzugsweise von 0,01-10 %, bezogen auf das Gewicht der Gesamtzusammensetzung, vorhanden sind.

13. Zusammensetzungen nach einem der Ansprüche 11 und 12, dadurch gekennzeichnet, daß das Lösungsmittel ein Polyalkohol, insbesondere ein Glykol, wie Propylenglykol ist.